# EUROPEAN PATENT SPECIFICATION

(11) **EP 0 278 973 B1**
(45) Date of publication and mention of the grant of the patent: **12.01.1994**
(21) Application number: 87905864.2
(22) Date of filing: 21.08.1987
(51) Int. Cl.: C02F 3/00

(54) **PROCESS FOR THE DEGRADATION OF COAL TAR CONSTITUENTS BY WHITE ROT FUNGI**
VERFAHREN ZUM ABBAU VON KOHLETEERBESTANDTEILEN MIT FAEULNISPILZEN
PROCEDE DE DEGRADATION DES CONSTITUANTS DU GOUDRON DE HOUILLE PAR L'UTILISATION DE MOISISSURES PUTREFACTRICES

(30) Priority: 22.08.1986 US 899000
(43) Date of publication of application: 24.08.1988
(73) Proprietor: THE BOARD OF TRUSTEES OF THE MICHIGAN STATE UNIVERSITY, East Lansing, Michigan 48824-1046 (US)
(72) Inventor: AUST, Steven, D., North Logan, UT 84321 (US); BUMPUS, John, A., North Logan, UT 84321 (US)
(74) Representative: Orr, William McLean
(86) International application number: US8702084
(87) International publication number: WO8801255

(56) References cited:
- US-A- 3 979 283
- Applied and Environmental Microbiology 43(5), May 1982, (Washington, DC), Cerniglia et al, "Glucoronide and Sulfate Conjugation in the Fungal Metabolism of Aromatic Hydrocarbons", pages 1070-1075
- Applied and Environmental Microbiology 44(1), July 82, (Washington DC, Cohen et al, "Degradation of coal by fungi polyporus versicolor and poria monticola", pages 23-27

## Description

The present invention relates to a process for the degradation of polycyclic aromatic compounds found in coal tar mixtures. In particular the present invention relates to the biodegradation of coal tar.

Most microorganisms are not able to cause complete biodegradation of polycyclic aromatic compounds. A few species of bacteria and fungi are able to cause extensive degradation of relatively simple polycyclic aromatic compounds (biphenyl and naphthalene, for example). On the other hand, polyaromatic compounds such as benzo(a)pyrene, a compound composed of five aromatic rings, is resistant to biodegradation by most, but not all microorganisms and is acknowledged to be a persistent environmental pollutant. Although some microorganisms are able to degrade certain polycyclic aromatic compounds, their usefulness in microbial waste treatment systems is often limited for the following reasons: (1) degradation is often not extensive, that is to say degradation does not always proceed to carbon dioxide as an end product of metabolism; (2) some metabolites may be even more toxic than the parent compound and as environmentally persistent and (3) many microorganisms used in waste treatment systems are not able to degrade a wide variety of organopollutants, and thus mixtures of microorganisms often have to be used instead of a single species.

The ability of a microorganism to degrade a broad spectrum of organopollutants is important in the treatment of sites contaminated with coal tar, a complex mixture of aromatic and polycyclic aromatic compounds that are by-products of the coking and coal gasification industries. For years coal tar wastes have been deposited in unlined dump sides where they have contaminated soils and ground water. The problem is to use microorganism to degrade the constituents found in coal tar.

Cohen (Applied as Environmental Microbiology, 44(1) 23-27, 1982) discloses that two species of basidiomycete fungi are capable of cultivation on lignite coal.

Preferred embodiments of the present invention provide a process for the microbial degradation of the constituents of coal tar and polycyclic aromatic constituents thereof. Further, preferred embodiments provide a process which is simple and economical.

The invention is further described by means of example but not in any limitative sense with reference to the accompanying drawings of which:

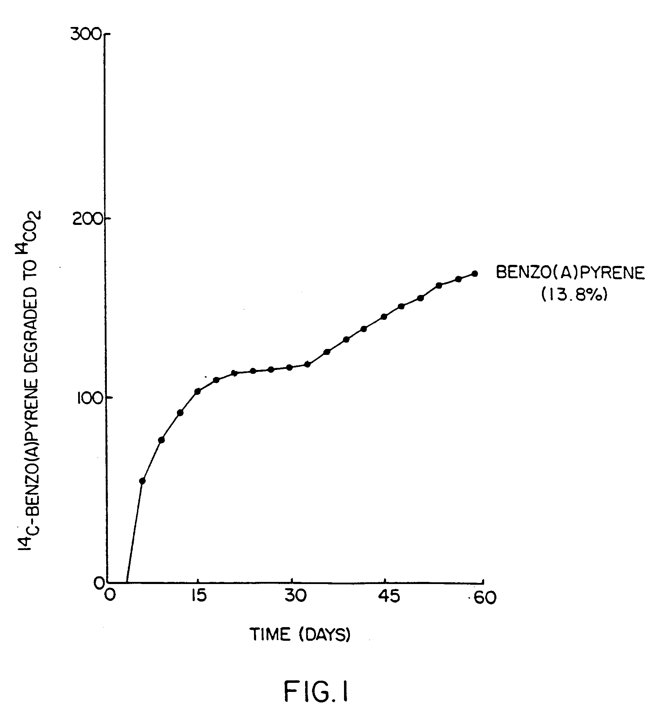

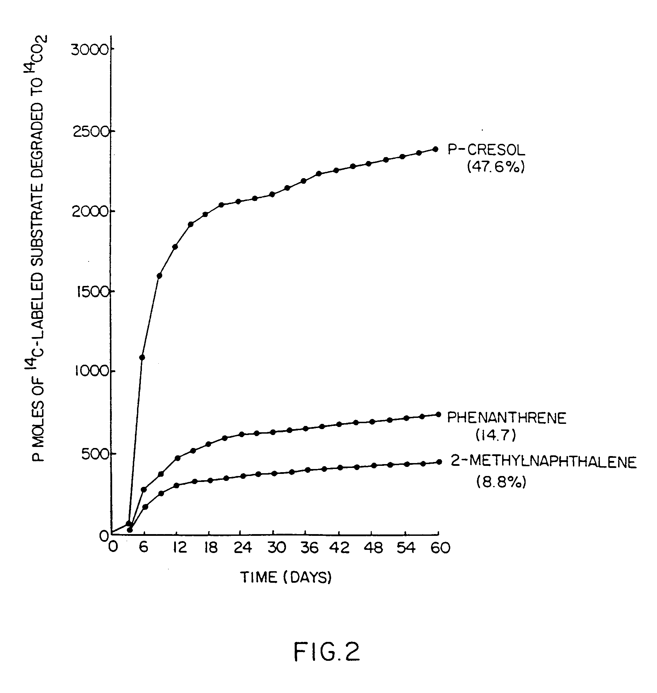

Figures 1 and 2 are graphs showing the ability of a white rot fungus (ie Phanerochaete chrysosnorium) to mineralise representative ¹⁴C-labelled compounds which are found in coal tar.

The present invention relates to a process for biodegrading coal tar or constituents thereof. The process comprising mixing of coal tar or polycyclic aromatic constituents found in coal tar with white rot fungi and appropriate nutrients under aerobic conditions. The mixture may also be supplemented by providing additional lignin degrading oxidases and hydrogen peroxidase. The coal tar constituents, including polycyclic aromatic compounds, are degraded to carbon dioxide.

Specifically this invention relates to the ability of white rot fungi (eg Phanerochaete chrysosporium) to degrade normally hard-to-degrade polycyclic aromatic compounds found in coal tar. At the same time, easier-to-degrade organopollutants such as single aromatic ring compounds will also be degraded by white rot fungi. Furthermore, because ¹⁴CO₂ has been found to be the ultimate degradation product in all cases tested, it is apparent that any potentially toxic intermediates from the degradation of the coal tar are also degraded. The process is very non-specific and capable of completely degrading a wide variety of organic molecules and thus is well suited for treatment of coal tar contaminated materials. Furthermore, because degradative enzymes are secreted by white rot fungi, treatment systems can consist of adding large amounts of these enzymes to effect initial oxidative degradation of the pollutants in the coal tar. Supplemental enzyme treatment can also take place in the presence of white rot fungi or other microorganisms

### Example 1

Because coal tar is a complex mixture containing well over 50 compounds, it was necessary to select representative compounds for evaluation. If these compounds are degraded, the rest will be degradable. The compounds studied and the reasons for their selection are listed below:
(1) p-cresol - This monocyclic aromatic compound is an abundant component found in coal tar. It is relatively water soluble and, therefore, represents a major threat to ground water. This compound is representative of one (1) ring compounds found in coal tar and is relatively easily degraded. It is noted that degradation of p-cresol alone by P chrysosporium has been demonstated by others.
(2) 2-methylnaphthalene - This compound is an abundant component found in coal tar. It is slightly water soluble, and, therefore, a threat to ground water. This compound along with naphthalene makes up greater than 70% of the organopollutants in some coal tar contaminated sites. This compound is representative of two ring polycyclic compounds found in coal tar.
(3) Phenanthrene - This polycyclic compound is found in coal tar and is a suspect carcinogenic compound. This compound is representative of three ring compounds found in coal tar. It is noted that degradation of this compound by P chrysosporium has not been reported by others.
(4) benzo(a)pyrene - This polycyclic aromatic compound is found in coal tar and is a carcinogenic compound. It is very difficult to degrade in the environment and it is representative of five ring compounds found in coal tar. It is noted that the inventors were the first to demonstrate degradation of this compound by P chrysosporium.

The degradation of various ¹⁴C-labelled coal tar constituents, ie p-cresol, phenanthrene, benzo(a)pyrene and 2-methylnaphthalene was accomplished separately in cultures of P chrysosporium as shown in Figures 1 and 2. Degradation, as assayed by ¹⁴CO₂ evolution was monitored for thirty (30) days at which time supplemental glucose was added and degradation was monitored for another 30 days (60 days total).

Each compound was incubated in agueous culture as described by Bumpus, et al in EP 192237. The culture medium was nutrient nitrogen deficient and was composed of 56 mM glucose, 1.2 mM ammonium tartrate, 20 mM dimethyl succinate buffer, pH 4.2-4.3. Cultures (10 ml) also contained trace amounts of thiamine and minerals as described by Kirk, T K et al Arch Microbiol 117, 227-285 (1977). The initial concentration of ¹⁴C-p-cresol, ¹⁴C-2-methylnaphthalene and ¹⁴C-pehnanthrene was 5 nmoles/culture. The initial concentration of ¹⁴C-benzo(a)pyrene was 1.25 nmoles/culture. Evolution of ¹⁴CO₂ from each coal tar constituent was monitored as described by Kirk et al.

As can be seen from Figures 1 and 2, the representative compounds are degraded up to the percentage levels indicated. The medium can be supplemented with more nutrient medium including particularly an additional carbon source and the process reinitiated until the degradation is complete. As can ben seen 2-methylnaphthalene is the most difficult to degrade and will therefore require repeated treatment.

The carbon source can be glucose, cellulose or any other compound which provides energy to the white rot fungus. The culture media for enabling the white rot fungus to degrade coal tar or derivatives thereof are well known to those skilled in the art.

The process can be performed in the field or in a reaction vessel where large amounts of liquid are used. It is referred to use a reaction vessel to prevent leaching into the ground water.

## Claims

1. A process for degrading polycyclic aromatic constituents of coal tar which comprises:
(a) providing a degradation resistant polycyclic aromatic constituent of coal tar having three or more rings mixed with fungal enzymes containing a lignin degrading oxygenase expressed by a white rot fungus in a growth medium containing a minimal nitrogen source level and with hydrogen peroxide under aerobic conditions; and
(b) degrading the polycyclic aromatic constituent of coal tar to form substantially less toxic degradation products.

2. The process of claim 1 wherein the enzymes and hydrogen peroxide are provided by a living fungus and wherein a carbon source enabling degradation of the polycyclic aromatic constituents of coal tar by the fungus is mixed with the polycyclic aromatic constituent of coal tar.

3. The process of claim 2 wherein the fungus has been grown in the growth medium.

4. The process of claim 3 wherein the growth medium includes thiamine and minerals for the fungus.

5. The process of claim 4 wherein the source of carbon is carbohydrate.

6. The process of claim 5 wherein the source of carbon is glucose.

7. The process of claim 1 wherein the polycyclic aromatic constituent of coal tar is mixed with soil from the environment and wherein the soil is mixed with the fungal enzymes and the hydrogen peroxide.

8. The process of claim 7 wherein the soil is mixed with the fungus which provides the fungal enzymes and hydrogen peroxide.

9. The process as claimed in claim 1 performed in a reaction vessel.

10. The process of claim 2 wherein the polycyclic aromatic constituent of coal tar is degraded to form nontoxic degradation products comprising water and carbon dioxide.

## Patentansprüche

1. Verfahren zum Abbau polyzyklischer aromatischer Kohleteerbestandteile, welches umfaßt:
a) ein Abbau-resistenter polyzyklischer aromatischer Kohleteerbestandteil mit drei oder mehr Ringen wird unter ärobischen Bedingungen bereitgestellt, der mit eine Lignin-abbauende Oxygenase enthaltenden Pilzenzymen, die aus einem weiß-roten Pilz in einem Wachstumsmedium gewonnen sind, welches einen minimalen Gehalt an Stickstoffguelle enthält, und mit Wasserstoffperoxid vermischt ist;
b) der polyzyklische aromatische Kohleteerbestandteil wird unter Bildung erheblich weniger giftiger Abbauprodukte abgebaut.

2. Verfahren nach Anspruch 1, bei welchem die Enzyme und das Wasserstoffperoxid durch einen lebenden Pilz bereitgestellt werden und bei welchem eine Kohlenstoffquelle, welche den Abbau des polyzyklischen aromatischen Kohleteerbestandteils durch den Pilz ermöglicht, mit dem polyzyklischen aromatischen Kohleteerbestandteil vermischt wird.

3. Verfahren nach Anspruch 2, bei welchem der Pilz in dem Wachstumsmedium gezüchtet worden ist.

4. Verfahren nach Anspruch 3, bei welchem das Wachstumsmedium Thiamin und Mineralien für den Pilz enthält.

5. Verfahren nach Anspruch 4, bei welchem die Kohlenstoffquelle Kohlehydrat ist.

6. Verfahren nach Anspruch 5, bei welchem die Kohlenstoffquelle Glukose ist.

7. Verfahren nach Anspruch 1, bei welchem der polyzyklische aromatische Kohleteerbestandteil mit Erde aus der Umgebung vermischt ist und die Erde mit den Pilzenzymen und dem Wasserstoffperoxid vermischt wird.

8. Verfahren nach Anspruch 7, bei welchem die Erde mit dem Pilz vermischt wird, welcher die Pilzenzyme und Wasserstoffperoxid bereitstellt.

9. Verfahren nach Anspruch 1, welches in einem Reaktionsbehälter durchgeführt wird.

10. Verfahren nach Anspruch 2, bei welchem der polyzyklische aromatische Kohleteerbestandteil unter Bildung nicht giftiger Abbauprodukte, welche Wasser und Kohlendioxid umfassen, abgebaut wird.

## Revendications

1. Procédé pour dégrader les constituants aromatiques polycycliques du goudron de houille comprenant :
(a) la fourniture d'un constituant aromatique polycyclique du goudron de houille résistant à la dégradation présentant trois cycles ou plus, mélangé à des enzymes fongiques contenant une oxygénase dégradant la lignine obtenues par un champignon de pourriture blanche dans un milieu de croissance contenant un niveau minimal en source d'azote et à du peroxyde d'hydrogène en conditions aérobies ; et
(b) la dégradation du constituant aromatique polycyclique du goudron de houille pour former des produits de dégradation sensiblement moins toxiques.

2. Procédé selon la revendication 1 dans lequel les enzymes et le peroxyde d'hydrogène sont fournis par un champignon vivant et dans lequel une source de carbone permettant la dégradation des constituants aromatiques polycycliques du goudron de houille par le champignon est mélangée avec le constituant aromatique polycyclique du goudron de houille.

3. Procédé selon la revendication 2, dans lequel le champignon s'est développé dans le milieu de croissance.

4. Procédé selon la revendication 3, dans lequel le milieu de croissance comprend de la thiamine et des minéraux pour le champignon.

5. Procédé selon la revendication 4, dans lequel la source de carbone est un hydrate de carbone.

6. Procédé selon la revendication 5, dans lequel la source de carbone est du glucose.

7. Procédé selon la revendication 1, dans lequel le composé aromatique polycyclique du goudron de houille est mélangé au sol environnant et dans lequel le sol est mélangé avec les enzymes fongiques et le peroxyde d'hydrogène.

8. Procédé selon la revendication 7, dans lequel le sol est mélangé avec le champignon qui fournit les enzymes fongiques et le peroxyde d'hydrogène.

9. Procédé selon la revendication 1, réalisé dans une cuve de réaction.

10. Procédé selon la revendication 2, dans lequel le constituant aromatique polycyclique du goudron de houille est dégradé pour former des produits de dégradation non toxiques comprenant de l'eau et du dioxyde de carbone.
